Europäisches Patentamt

(19) European Patent Office

Office européen des brevets

(11) Veröffentlichungsnummer: **0 262 445**
**B1**

(12) **EUROPÄISCHE PATENTSCHRIFT**

(45) Veröffentlichungstag der Patentschrift:
**22.11.90**

(21) Anmeldenummer: **87112875.7**

(22) Anmeldetag: **03.09.87**

(51) Int. Cl.⁵: **G01N 33/52**

(54) **Mehrschichtiger Testträger.**

(30) Priorität: **12.09.86 DE 3630999**

(43) Veröffentlichungstag der Anmeldung:
**06.04.88 Patentblatt 88/14**

(45) Bekanntmachung des Hinweises auf die Patenterteilung:
**22.11.90 Patentblatt 90/47**

(84) Benannte Vertragsstaaten:
**AT BE CH DE ES FR GB GR IT LI LU NL SE**

(56) Entgegenhaltungen:
**EP-A- 0 182 373**
**DE-A- 3 130 749**
**DE-A- 3 323 973**
**US-A- 3 933 594**

(73) Patentinhaber: **BOEHRINGER MANNHEIM GMBH,
Sandhofer Strasse 116, D-6800 Mannheim 31(DE)**

(72) Erfinder: **Deneke, Ulfert, Dr. rer. nat., Birkenweg 5,
D-6149 Rimbach-Zotzenbach(DE)**
Erfinder: **Nagel, Rolf, Am Bildstock 51,
D-6842 Bürstadt(DE)**
Erfinder: **Rothe, Dr. rer. nat. Anselm,
Tiefenklingerweg 21, D-6943 Birkenau(DE)**
Erfinder: **Freitag, Dr. rer. nat. Helmut, An der
Ziegelhütte 13, D-6940 Weinheim(DE)**

## Beschreibung

Die Erfindung betrifft einen mehrschichtigen Testträger zur analytischen Bestimmung eines Bestandteils einer flüssigen Probe, insbesondere einer Körperflüssigkeit, mit einer ersten Tragschicht und einer zweiten Tragschicht, von denen wenigstens eine durchsichtig ist, wobei auf die erste Tragschicht eine absorbierende Schicht und auf die zweite Tragschicht eine mindestens ein Reagenz enthaltende Reagenzschicht aufgebracht ist und wobei die Tragschichten so angeordnet und befestigt sind, daß die von den Tragschichten abgewandten Oberflächen der absorbierenden Schicht und der Reagenzschicht in Kontakt zueinander gebracht und gegeneinander gedrückt werden können.

Testträger haben bei der Analyse von Flüssigkeiten eine erhebliche Bedeutung erlangt. Insbesondere in der klinischen Chemie, die sich mit der qualitativen und quantitativen analytischen Bestimmung von Bestandteilen von Körperflüssigkeiten (insbesondere Blut und Urin) befaßt, werden ihre Vorteile sehr geschätzt. Während die klassischen, mit flüssigen Reagenzien arbeitenden Methoden eine Vielzahl von Handhabungsschritten erfordern, zeichnen sich analytische Bestimmungen mit Hilfe von Testträgern durch eine äußerst einfache Handhabung aus. Bei der Analyse von Urin werden im allgemeinen Testträger in Form von Teststreifen eingesetzt, die in die Probe kurz eingetaucht und danach entweder visuell oder apparativ ausgewertet werden. Bei der Blutanalyse wird ein Tropfen Serum oder – bei einer besonders fortschrittlichen Art von Testträgern – Blut an einer vorbestimmten Stelle des Testträgers aufgegeben.

Die Reaktion der Flüssigkeit mit den auf dem Testträger vorhandenen Reagenzien führt zu einer wahrnehmbaren Veränderung des Testträgers, meist zu einer Veränderung seiner Farbe, z.B. durch Bildung oder Freisetzung eines Farbstoffes. Die Reaktion kann aber auch zur Entstehung oder Änderung einer fluoreszierenden oder in anderer Weise optisch oder in sonstiger Weise wahrnehmbaren Substanz führen. Testträger für die Bestimmung von Blutbestandteilen werden überwiegend apparativ mit Hilfe entsprechender Geräte ausgewertet. Die vorliegende Erfindung richtet sich insbesondere auf Testträger zur quantitativen Bestimmung von Flüssigkeitsbestandteilen mit Hilfe eines Reflexierungsphotometers oder eines anderen optischen Auswertegerätes.

Testträger werden in verschiedenen Ausführungsformen hergestellt. In der Anfangszeit der Entwicklung waren nahezu ausschließlich Teststreifen gebräuchlich, die eines oder mehrere Testfelder tragen. Die Testfelder bestanden in der Anfangszeit überwiegend aus Papier oder einem ähnlichen porösen, aufgrund der Kapillarkräfte zwischen den einzelnen Fasern saugfähigen Material. Später wurden die Testfelder zunehmend auch in Form von polymeren Schichten ausgebildet, bei denen in einer Matrix, die auch als Filmschicht bezeichnet werden kann, Reagenzien eingebettet sind. Derartige Reagenzfilme kann man in zwei grundsätzlich verschiedene Typen unterteilen, nämlich einerseits poröse Schichten, die aufgrund von Kapillarkräften die Probenflüssigkeit in sich aufnehmen und andererseits quellfähige Filme, in die die Probenflüssigkeit, die im Fall von Körperflüssigkeiten immer wäßriger Natur ist, langsam aufgrund der Aufnahme des Wassers in die struktur des Films eindringt. Beiden Typen ist gemeinsam, daß die Reagenzien in dem Film eingebettet sind und auch bei Ablauf der Reaktion darin bleiben, d.h. die Probe dringt in den Film ein, in welchem die Reaktion mit den Reagenzien abläuft.

Auch bezüglich der äußeren Gestaltung sind mittlerweile sehr verschiedene Testträgertypen entwickelt worden. So werden beispielsweise quadratische Plättchen verwendet, die, ähnlich einem photographischen Dia, in einem Rahmen aus Karton ein ebenfalls quadratisches Testfeld mit dem Reaktionsfilm enthalten. Andere Hersteller bevorzugen einen dem klassischen Teststreifen ähnlichen Aufbau.

In der Deutschen Patentschrift 3 130 749 ist ein Testträger der eingangs bezeichnenden Art beschrieben. Die erste Tragschicht wird in diesem Fall durch eine längliche Basisfolie wie bei einem Teststreifen gebildet. Darauf ist die absorbierende Schicht angeordnet. An der Basisfolie ist eine rechteckige Deckfolie, die ebenso breit ist wie die Basisfolie, aber erheblich kürzer als diese, mit einer Kante befestigt. Die Befestigungstelle liegt in der Nähe des Endes der absorbierenden Schicht. Auf der der Basisfolie zugewandten Seite der Deckfolie befindet sich eine Reagenzschicht, insbesondere ein Reagenzfilm. Die Deckfolie mit dem Reagenzfilm bildet eine Klappe, wobei die Verbindung mit der Basisfolie so gestaltet ist, daß die Klappe im Ruhezustand nicht die absorbierende Schicht berührt. Dadurch ist es möglich, den zeitlichen Ablauf einer Reaktion in zwei Schritte zu unterteilen. Die Probe wird zunächst auf die absorbierende Schicht aufgebracht, wobei sie mit gegebenenfalls in dieser Schicht inkorporierten Reagenzien reagieren kann. Zu einem bestimmten Zeitpunkt wird die Deckfolie mit ihrer Reagenzschicht auf die Basisfolie aufgedrückt. Durch diesen vollflächigen Kontakt kann die in der absorbierendne Schicht enthaltene Probe in die Reagenzschicht der Klappe übertreten. Dort findet eine weitere Reaktion statt, die zu einem nachweisbaren Signal, beispielsweise zu einem Farbumschlag führt.

Mit Hilfe der heute erhältlichen mehrschichtigen Testträger können auch sehr aufwendige Bestimmungen durchgeführt werden. Da die verschiedenen Reagenzien, die hierfür erforderlich sind, häufig in verschiedenen, voneinander getrennten Schichten untergebracht werden müssen, haben derartige Testträger häufig verhältnismäßig viele Schichten, die alle von der aufgegebenen Probe durchfeuchtet werden müssen. Da andererseits die Probenmenge, aus der der Test durchgeführt wird, möglichst gering gehalten werden soll (ca. 30 μl -50 μl) werden die einzelnen Schichten möglichst dünn gemacht. Hierfür eignen sich insbesondere die erwähnten Filmschichten, die sich gegenüber Schichten auf Papierbasis darüber hinaus durch eine besonders gute Homogenität auszeichnen. Die Verwendung sehr dünner Schichten bringt allerdings neue Probleme mit sich, u. a. weil die Gefahr besteht, daß Reagenzien, wenn sie von der Probenflüssigkeit gelöst worden sind, aus einer Schicht herauswandern und in eine andere Schicht eindringen. Wenn beispielsweise in einer Reagenzschicht aufgrund einer Reaktion zwischen einem Bestandteil der Probe oder einem Folgeprodukt einer Vorreaktion eine Farbe gebildet wird, so ist es wesentlich, daß die so gebildete Farbsubstanz in derjenigen Schicht verbleibt, in der sie gemessen werden soll.

In der europäischen Patentanmeldung 0 182 373 ist ein mehrschichtiger Testträger beschrieben, der neben einer Auftragsmatrix (3), einem Transportvlies (5) und einer Abdeckfolie (8), ein Reagenzträgermaterial (7) enthält, das aus einem saugfähigen oder quellfähigen Material hergestellt werden kann. Lösliche filmbildende Materialien sind als mögliche Reagenzträgermaterialien erwähnt. Eine diffus reflektierende Tragschicht wird in dieser Anmeldung nicht offenbart. Im Gegenteil wird auf Seite 7, 2. Absatz, darauf hingewiesen, daß Basis- und/oder Abdeckfolie vorzugsweise transparent sind.

Ausgehend von dieser Problematik besteht ein Bedarf nach Testträgern, die mit einer geringen Probenmenge auskommen, dennocn einen mehrschichtigen Aufbau ermöglichen und das Problem der unkontrollierten Diffusion von Reagenzbestandteilen aus einer Schicht in eine benachbarte Schicht vermeiden.

Dieses Bedürfnis wird bei einem Testträger der eingangs bezeichnetn Art dadurch glöst, daß die Reagenzschicht in der Probenflüssigkeit im wesentlichen rückstandsfrei löslich ist und die Aufnahmefähigkeit der absorbierenden Schicht für die Probenflüssigkeit so groß ist, daß eine darin absorbierte Flüssigkeitsmenge ausreicht, um die Reagenzschicht rückstandsfrei zu lösen, so daß in der Reagenzschicht enthaltene Substanzen aus der Reagenzschicht in die flüssigkeitsabsorbierende Schicht übertreten, wenn beide Schichten gegeneinander gedrückt werden, und daß die flüssigkeitsabsorbierende Schicht bezüglich ihrer optischen Absorptionseigenschaften und ihrer Schichtdicke so gewählt wird, daß sie vom Meßlicht eines üblichen remissionsphotometrischen Auswertegerätes durchdrungen wird und daß eine der Tragschichten für das Meßlicht diffus reflektierend wirkt.

Der Testablauf unterscheidet sich bei einem erfindungsgemäßen Testträger im Vergleich zu dem der Deutschen Patentschrift 3 130 749 also grundsätzlich dadurch, daß die Reaktion zwischen den Reagenzien der Reagenzschicht und der Probe nicht in der Reagenzschicht, sondern in der absorbierenden Schicht abläuft.

Es ist wesentlich, daß die Reagenzschicht sich schnell und im wesentlichen rückstandsfrei in der Probenflüssigkeit auflöst. Sie enthält daher bevorzugt keine Pigmente. «Im wesentlichen» rückstandsfrei ist dabei in Relation zur gewünschten Meßgenauigkeit zu verstehen. Bleiben auf der zweiten Tragschicht nicht aufgelöste Teile der Reagenzschicht zurück, so führt dies zu einer Verfälschung des Meßergebnisses. Die Auflössung muß daher so vollständig sein, daß die gewünschte Meßgenauigkeit erreicht wird.

Weiterhin ist das in der flüssigkeitsabsorbierenden Schicht speicherbare Flüssigkeitsvolumen von Bedeutung. Es wird bestimmt von der Dicke der Schicht und ihrem Absorptionsvermögen für die Flüssigkeit.

Die in der Reagenzschicht enthaltenen Reagenzien sollen vollständig in die absorbierende Schicht eindringen. Um dieses zu erleichtern, sollte die Reagenzschicht dünner als die flüssigkeitsabsorbierende Schicht, bevorzugt höchstens halb so dick wie diese ausgebildet sein.

Wesentlich für den Erfolg ist die Auswahl eines geeigneten Materials, aus dem die Reagenzschicht gebildet ist. Gemäß bevorzugter Ausführungsform ist die Reagenzschicht als Film aus einem hochmolekularen, polymeren, reversibel in Wasser löslichen Material ausgebildet. Als Filmbildner eignen sich besonders Materialien, die auch als Binde- oder Quellmittel oder als Verdicker eingesetzt werden. Beispielhaft genannt seien Polyvinylpyrrololidon, Polyvinylalkohol, Methylcellulose, Methylhydroxyäthylcellulose, Hydroxyäthylcellulose und Alginate, insbesondere Natriumalginat.

Als besonders geeigneter Filmbildner für die Reagenzschicht hat sich Xanthan erwiesen. Dies ist ein natürliches hochmolekulares Polysaccharid das von der Kelco-Division der Firma Merck & Co., USA, unter den Markennamen Keltrol und Kelzan vertrieben wird. Es wird mit Hilfe des Mikroorganismus Xanthomonas campestris hergestellt. Die Zellwand dieser Mikroorganismen ist außen von Xanthan umgeben. Das Material wird durch entsprechende Reinigungsverfahren aus Kulturen des genannten Organismus gewonnen. Die Struktur enthält enthält drei verschiedenen Monosaccharide, nämlich Mannose, Glucose und Glucuronsäure. Nähere Informationen sind insbesondere aus Firmenpublikationen des genannten Unternehmens erhältlich.

Die flüssigkeitsabsorbierende Schicht kann aus einer Vielzahl von Materialien bestehen, die die oben erwähnten Anforderungen an ihre Aufnahmefähigkeit für die Probenflüssigkeit erfüllen. Besonders geeignet sind Papiere, Vliese und solche Filmschichten, die aufgrund einer offenen, zahlreiche Kapillarspalten aufweisenden Struktur ein erhebliches Aufnahmevermögen für Flüssigkeit haben.

Wie erwähnt führt die Reaktion zwischen der in der absorbierenden Schicht enthaltenen Probenflüssigkeit, die gegebenenfalls schon vorgelagerte Vorreaktionen durchlaufen hat, mit den Reagenzien der Reagenzschicht zu einem nachweisbaren Signal, insbesondere zu einer Farbbildung. Andere Möglichkeiten nachweisbarer Signale wurden erwähnt. Im folgenden wird jedoch stellvertretend, ohne Beschränkung der Allgemeinheit, nur auf die Bildung einer Farbe und die entsprechende photometrische Messung mit Hilfe eines Reflexionsphotometers eingegangen.

Beim erfindungsgemäßen Testträger treten die Reagenzien wie erwähnt in die flüssigkeitsabsorbierende Schicht über, so daß dort und nicht in der Reagenzschicht selbst die Farbbildung stattfindet. Überraschenderweise hat sich gezeigt, daß durch die Tatsache, daß die flüssigkeitsabsorbierende Schicht verhältnismäßig dick und bevorzugt aus einem fasrigen Material gebildet ist, die qualität der optischen Messung nicht beeinträchtigt wird. Es ergibt sich sogar eine besonders gute Meßgenauigkeit, wenn die flüssigkeitsabsorbierende Schicht aus einem Material mit einem verhältnismäßig geringen optischen Absorptionsvermögen hergestellt ist, so daß sie vom Meßlicht eines entsprechenden Remissionsphotometers durchdrungen wird. In diesem Fall muß mindestens eine der Tragschichten, bevorzugt die Basisfolie, diffus reflektierend sein. Dadurch wird das Meßlicht durch die flüssigkeitsabsorbierende Schicht zurückreflektiert und von dem Meßempfänger des Reflektionsphotometers nachgewiesen. Dadurch, daß das Meßlicht die volle Schichstärke der flüssigkeitsabsorbierenden Schicht zweimal durchdringt, ist die der Messung zugrundeliegende optische Schichtdicke verhältnismäßig groß, was zu einer ausgezeichneten Empfindlichkeit der Messung führt. Im Gegensatz dazu mußten bei den vorbekannten Teststrägern gemäß der Deutschen Patentschrift 3 130 749 bevorzugt Reagenzschichten mit einem hohen Pigmentanteil eingesetzt werden, damit weitgehend nur die Farbbildung nahe der Deckfolie zugewandten Oberfläche der Reagenzschicht beobachtet wurde. Dies war notwendig, um Meßfehler aufgrund des Wegdiffundierens von Farbstoffen zu vermeiden, führte aber zu einer geringen optischen Schichtstärke und damit zu einem Verlust an Empfindlichkeit.

Die Erfindung wird im folgenden anhand eines in den Figuren schematisch dargestellten Ausführungsbeispiel näher erläutert. Es zeigen:

Figur 1 einen Längsschnitt durch einen erfindungsgemäßen Testträger in einem Zustand, in dem die Reagenzschicht nicht an die flüssigkeitsabsorbierende Schicht gedrückt ist und

Figur 2 einen Testträger nach Figur 1, bei dem die Reagenzschicht gegen die flüssigkeitsabsorbierende Schicht gedrückt ist.

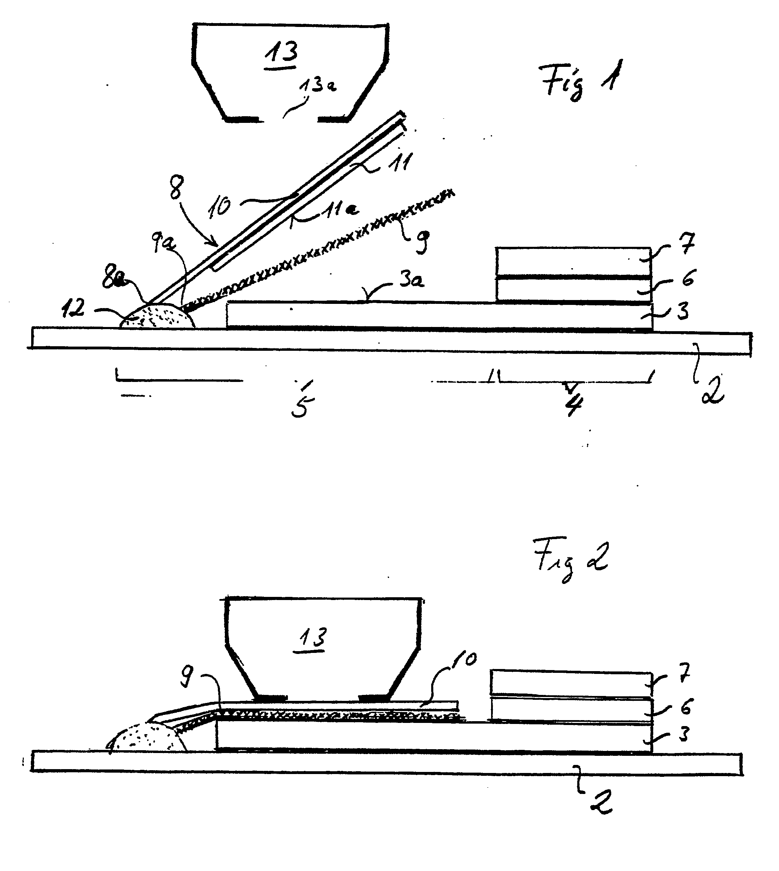

Der in Figur 1 dargestellte Testträger 1 hat eine Basisfolie 2, die als Tragschicht für eine darauf vollflächig anliegende absorbierende Schicht 3 dient. die Basisfolie 2 ist schmal und langgestreckt wie bei einem Teststreifen. Die darauf angeordneten Schichten haben die gleiche Breite wie die Basisfolie 2, erstrecken sich aber nur über einen Teil von deren Länge.

Der gesamte Testbereich des Testträgers 1 läßt sich in einen Probenaufgabenbereich 4 und in einen Auswertungsbereich 5 unterteilen. In Probenaufgabenbereich 4 ist oberhalb der absorbierenden Schicht 3 eine Vorreagenzschicht 6 und eine Plasmaabtrennungsschicht 7 vorgesehen. Diese beiden Schichten überdecken nur einen Teil der absorbierenden Schicht 3.

Im Answertungsbereich hat der Testträger eine insgesamt mit 8 bezeichnete Klappe und ein Reagenzträger 9. Die Klappe 8 und der Reagenzträger 9 sind jeweils rechteckig und haben die Breite der Tragschicht 2. Mit einer ihrer kurzen Kanten 8a bzw. 9a sind sie derartig an der Basisfolie 2 befestigt, daß sie im entspannten Zustand nicht in Kontakt zu der absorbierenden Schicht 3 stehen, jedoch durch äußeren Druck mit der Schicht 3 in Kontakt gebracht werden können.

Die Klappe 8 besteht aus einer Deckfolie 10 und einer darauf beschichteten Reagenzschicht 11. In der Darstellung bedeckt die Reagenzschicht 11 die Deckfolie 10 nur über einen Teil ihrer Länge. Aus Fertigungsgründen kann es jedoch zweckmäßig sein, die gesamte Deckfolie 10 auf der der absorbierenden Schicht 3 zugewandten Oberfläche mit einer Reagenzschicht 11 zu beschichten.

Der Reagenzträger 9 hat eine für Flüssigkeit offene Verbundstruktur, so daß er den Flüssigkeitsaustausch zwischen der Reagenzschicht 11 und der absorbierenden Schicht 3 nicht behindert. Er besteht bevorzugt aus einer Trägermatrix aus offenem, sehr dünnen Gewebe oder Papier, auf die lösliche Reagenzeien imprägniert sind. Die Trägermatrix muß einerseits fest genug sein, um im trockenen Zustand bei der Lagerung und Handhabung des Testträgers nicht beschädigt zu werden. Andererseits muß sie so dünn und offen in ihrer Struktur sein, daß die optische Messung nicht in einem die Meßgenauigkeit beeinträchtigenden Maß gestört wird. Als geeignet hat sich insbesondere Teebeutelpapier erwiesen.

Zur Durchführung einer Analyse wird ein Tropfen von beispielsweise 30 µl Blut auf die Plasmaabtrennungsschicht 7 aufgebracht. Er sickert durch diese Schicht, wobei die Erythrozyten im Blut abgetrennt werden (näheres ist der US-Patentschrift 4 477 575 zu entnehmen). Das so gebildete Plasma gelangt in die Vorreagenzschicht 6, wo er eines oder mehrere darin enthaltene Reagenzien löst, mit denen die Probe reagieren kann. Aus der Vorreagenzschicht 6 gelangt die Probe in die absorbierende Schicht 3 und wird von dieser in Längsrichtung des Testträgers in den Auswertungsbereich 5 transportiert. Zu diesem Zweck hat die absorbierende Schicht 3 Kapillartransporteigenschaften in dieser Richtung, also in Richtung ihrer Flächenausdehnung.

Die absorbierende Schicht 3 und die Plasmaabtrennungsschicht 7 sind bevorzugt aus Glasfaser hergestellt (vgl. US-Patentschrift 4 477 575). Sie kann jedoch auch aus einem anderen Material, das die oben angegebenen Bedingungen erfüllt, hergestellt sein.

Im Auswertungsbereich 5 steht in dem zuvor beschriebenen Betriebszustand Plasma in der absorbierenden Schicht 3 zur Verfügung, wobei gegebenenfalls schon eine Vorreaktion mit dem Vorreagenz aus der Schicht 6 abgelaufen ist. Zu einem genau definierten Zeitpunkt kann nun durch Herunterdrücken der Klappe 8 auf die absorbierende Schicht 3 eine weitere Reaktion eingeleitet werden. Figur 2 zeigt den

EP 0 262 445 B1

Zustand in dem die absorbierende Schicht 3 und die Reagenzschicht 11 mit ihren einander zugewandten Oberflächen 3a bzw. 11a in einem einen Flüssigkeitsaustausch ermöglichenden Kontakt zueinander stehen.

Bei der in den Figuren dargestellten Ausführungsform wird der Druck auf die Klappe 8 von dem Meßsystem 13 eines entsprechenden Auswertegerätes ausgeübt. Zu dem genannten definierten Zeitpunkt, zu dem die Klappe 8 angedrückt werden soll, bewegt sich das Meßsystem 13 aus der in Figur 1 dargestellten Position schräg nach rechts unten, bis es auf der Deckfolie 10 aufliegt und die Klappe 8 gegen die absorbierende Schicht 3 drückt. Nähere Einzelheiten für eine geeignete Konstruktions eines optischen Meßsystem sind der US-Patentschrift 4 523 853 zu entnehmen. eine geeignete Konstruktion für die Halterung des Testträgers und das Andrücken der Klappe sind Gegenstand der Europäischen Patentanmeldung mit der Publikationsnummer 129 220.

Nach dem Andrücken der Reagenzschicht 11 auf die absorbierende Schicht 3 löst die Probenflüssigkeit die Reagenzschicht 11 auf und die darin enthaltenen Reagenzien gelangen in die absorbierende Schicht 3. Die auf dem Reagenzträger 9 imprägnierten Reagenzien werden von diesem abgelöst und gelangen ebenfalls in die absorbierende Schicht 3. Dort läuft daraufhin eine Reaktion ab, die zu einer für die gewünschte analytische Bestimmung charakteristischen Verfürbung führt, die sich mit dem Meßsystem 13 reflexionsphotometrisch nachweisen läßt.

Zu diesem Zweck wird Meßlicht durch das Fenster 13a des Meßsystems 13 und die Deckfolie 10 der Klapper 8 sowie durch den Reagenzträger 9 in die absorbierende Schicht 3 eingestrahlt. Falls die absorbierende Schicht 3 stark pigmentiert ist, wird das Meßlicht nach mehr oder weniger tiefem Eindrigen in die absorbierende Schicht 3 diffus zurückreflektiert und von dem Meßsystem 13 in bekannter Weise nachgewiesen. Besonders bevorzugt ist das optische Absorptionsvermögen der absorbierenden Schicht 3 jedoch verfältnismäßig gering, so daß das Meßlicht die gesamte Schichtstärke durchdringt und erst von der in diesem Fall stark pigmentierten Basisfolie 2 diffus reflektiert wird. Dadurch steht die gesamte Schichtstärke der absorbierenden Schicht 3 für die optische Auswertung zur Verfügung, was, wie oben erwähnt, zu einer Verbressung der Meßgenauigkeit führt.

Bei der dargestellten Ausführungsform ist die die absorbierende Schicht 3 tragende erste Tragschicht eine verhältnismäßig steife Basisfolie von etwa 200 µm -600 µm Stärke und die zweite, die Reagenzschicht 11 tragende Tragschicht eine verhältnismäßig flexible Deckfolie von etwa 100 µm - 250 µm Stärke. Diese Anordnung ist für die Zwecke der Erfindung besonders bevorzugt, weil sich die flexible Deckfolie gut und glatt auf die absorbierende Schicht 3 andrücken läßt.

Weiterhin ist bei der dargestellten Ausführungsform die Deckfolie 10 durchsichtig und die Basisfolie 2 undurchsichtig. Dies ist notwendig, wenn, wie im dargestellten Ausführungsbeispiel von der Seite der Deckfolie her gemessen werden soll, was insbesondere dann zweckmäßig ist, wenn das Meßsystem gleichzeitig zum Andrücken der Klappe 8 dient.

Im Rahmen der Erfindung liegen jedoch auch andere Gestaltungsmöglichkeiten, beispielsweise kann die Basisfolie 2 durchsichtig und die Deckfolie 10 pigmentiert sein, wobei dann von der Seite der Basisfolie her, also in Figur 1 von unten, gemessen wird.

Für die Genauigkeit, der mit einem erfindungsgemäßen Testträger durchgeführten Analysen ist wesentlich, daß sich die Reagenzschicht ausreichend schnell und vollständig löst, so daß die Reagenzien in kurzer Zeit gleichmäßig in die flüssigkeitsabsorbierende Schicht eindringen und die optische Messung durch Reste der Reagenzschicht nicht wesentlich gestört wird. Um aus einer größeren Zahl von als geeignet erscheinenden Materialien, aus denen die Reagenzschicht gebildet werden kann, ein geeignetes Material auszuwählen, ist folgendes Auswahlverfahren geeignet:

Es werden wäßrige Lösungen der zu prüfenden Substanz in solchen Konzentrationen hergestellt, daß gut beschichtbare Viskositäten von ca. 30 – 50 mPasec resultieren. Den Lösungen werden pro 100 ml 100 mg Tween® 20 (ein Netzmittel, das von E. Merck, Darmstadt, Bundesrepublik Deutschland, vertrieben wird) und 60 mg des Farbstoffes Patentblau V (ebenfalls Hersteller Merck, Darmstadt) zugegeben. Die Lösungen werden in einer Naßfilmdicke von 0,1 mm auf eine 0,2 mm dicke Polycarbonatfolie beschichtet und bei 60°C getrocknet.

Die Eignung der so hergestellten Filmschicht für den erfindungsgemäßen Zweck läßt sich prüfen, indem man 30 µl Serum auftropft. Bei einer besonders gut geeigneten Filmschicht, insbesondere bei Verwendung von Xanthan als Filmbildner, breitet sich der Tropfen rasch flach aus. Der Farbstoff löst sich sofort und gleichmäßig.

Bei Verwendung anderer, weniger geeigneter Substanzen als Filmbildner breitet sich der Tropfen weniger rasch aus, d.h. die Flüssigkeit bleibt zunächst halbkugelförmig stehen. Der Farbstoff löst sich nur mit Verzögerung, und es bleiben optisch erkennbare Schlieren. Mit einer derartigen Substanz als filmbildendem Material wird zwar auch noch ein brauchbares Ergebnis erreicht, es werden aber keine gehobenen Ansprüche an die Meßgenauigkeit erfüllt.

Zu dieser zweiten, für die Erfindung zwar geeigneten, aber weniger bevorzugten Gruppe gehören die in der folgenden Tabelle 1 aufgelisteten Substanzen außer Xanthan. Zu jeder Substanz wird in Gewichtsprozent der Gehalt in der Lösung angegeben, der notwendig ist, um die ebenfalls angegebene Viskosität einzustellen, aus der sich beispielsweise eine erfindungsgemäße Reagenzschicht beschichten läßt.

| Tabelle 1: | Gehalt in der Beschichtungslösung (in Gew. %) | Viskosität ( mPasec) |
|---|---|---|
| Polyvinylpyrrolidon, Kollidon® K 17, | 30 | 30 |
| Polyvinylalkohol, Moviol 18-88, | 4,5 | 35 |
| Moviol 26-88 | 4 | 35 |
| Methylcellulose, Tylose 6000, | 0,5 | 40 |
| Tylose 30.000 | 0,5 | 45 |
| Methylhydroxyäthylcellulose, Tylose MH 2000 X P, | 1 | 45 |
| Hydroxyäthylcellulose, Natrosol 250 G, | 2 | 40 |
| Na-Alginat, Kelcoalginat, | 0,6 | 40 |
| Xanthan-Gum, Keltrol®, | 0,7 | 40 |

Die Substanzen wurden von folgenden Herstellern bezogen:
Nr. 1 BASF AG, Bundesrepublik Deutschland
Nr. 2 – 6 Hoechst AG, Bundesrepublik Deutschland
Nr. 7 Hercules, Bundesrepublik Deutschland
Nr. 8, 9 Kelco Division von Merck & Co., USA
Im folgenden werden zwei Beispiele für Ausführungsformen der Erfindung wiedergegeben.

Beispiel 1: Testträger zur Bestimmung von Amylase in Blut entsprechend Abbildung 1.

a) Herstellung der Reagenzschicht 11 aus

5 g einer 0,3%igen Lösung von Xanthan-Gum, Keltrol® F, in 0,1 M Phosphat-Puffer, pH 7
1000 U a-Glucosidase, (Hersteller: Boehringer Mannheim GmbH, Bundesrepublik Deutschland)
50 mg Tween® 20 (Netzmittel)
50 mg Indolyl-a-maltoheptaosid (Farbbildendes Substrat)

wird eine Beschichtunsgmasse hergestellt und mit 0,1 mm Naßfilmdicke auf eine 0,1 mm dicke durchsichtige Polycarbonatfolie (als Deckfolie 10) beschichtet und 15 Minuten bei 50°C getrocknet.
Ein alternativer Reagenzfilm enthält bei sonst identischer Zusammensetzung anstelle der Xanthan-Gum-Lösung eine 0,2%ige Lösung von «Tylose 6000» in Wasser.

b) Herstellung des Reagenzträgers 9

Eine Lösung von
60 mg 4-(N-Morpholino)-2-methoxy-phenyldiazoniumsalz (Farbverstärker)
0,5 g Phosphorsäuretrimorpholid (Stabilisator),
10 ml Wasser

wird auf ein monofiles Polyamidgewebe Nylon 20 HC der Firma Schweizer Seidengaze Fabrik / Thal, Schweiz, getränkt und 10 Minuten bei 30°C getrocknet.
Die Vorreaktionsschicht 6 ist bei diesem Test nicht vorhanden.

c) Meßergebnisse

Die Auswertung der Farbbildung wurde mit Hilfe des Gerätes "Reflotron" der Boehringer Mannheim GmbH, Bundesrepublik Deutschland, vorgenommen. Als Probe wurde amylasehaltiges Humanserum eingesetzt. Die Auswertung erfolgte über empirisch ermittelte Funktionskurven in U/1 (Units pro Liter).

| Soll (U/l) | je 15 Meßwerte mit Tylose haltigem Film | | je 15 Meßwerte mit Xanthan-haltigem Film | |
|---|---|---|---|---|
| | Mittelwert (U/l) | Variations-coeffizient VK (%) | Mittelwert (U/l) | Variations-coeffizient VK(%) |
| 152 | 149 | 10,4 | 154 | 4,08 |
| 290 | 281 | 5,8 | 293 | 2,48 |
| 741 | 732 | 7,4 | 746 | 1,39 |
| 1250 | 1177 | 5,9 | 1279 | 2,35 |

Man erkennt, daß Meßergebnisse des Xanthan-haltigen Films deutlich besser sind. Sie genügen auch sehr hohen Ansprüchen in der klinischen Chemie. Die Meßergebnisse des Tylose-haltigen Films genügen dagegen nicht ganz so hohen Genauigkeitsansprüchen. Je nach Einsatzzweck kann aber auch mit diesem Film erreichte Genauigkeit (beispielsweise für semiquantitative Bestimmungen) ausreichend sein.

Zusätzlich zur reflexionsphotometrischen Messung wurden die Testträger nach der Messung visuell beurteilt. Dabei zeigte sich, daß die unter der Deckfolie 10 erkennbare absorbierende Schicht beim Xanthan-Film gleichmäßig blau eingefärbt war, während der Tylose-haltige Film eine leichte Schlierenbildung mit lokalen Fehlfarben zeigte. Beim Abheben der Klappe mit einer Pinzette waren im Falle der Xanthan-Schicht keinerlei nicht gelöste Überreste unter der Klappe erkennbar. Die Tylose-Schicht war zwar auch weitgehend gelöst, jedoch waren offenbar geringe Reste zurückgeblieben, was sich durch Fadenziehung bemerkbar machte.

Biespiel 2: Testträger (gemäß Abb. 1) zur Bestimmung des niedrig konzentrierten Parameters Bilirubin im Blut.

Vergleich zwischen einem erfindungsgemäßen Testträger und einem Testträger gemäß dem Stand der Technik.

a) Herstellung eines Reagenzfilms gemäß dem Stand der Technik (anstelle der Reagenzschicht 11)

aus
0,4 Citronensäure
10 g Polyvinylpropionat-Dispersion 50%ig (Propiofan; BASF AG, Bundersrepublik Deutschland)
15 g Hydroxyäthylcellulose-Lösung 1%ig (Verdicker; Natrosol 250 G, Hercules)
0,3 g Triton® X 100 (Netzmittel; E. Merck, Darmstadt, Bundersrepublik Deutschland)
9,5 g Kieselgur
150 mg 2,5-Di-Chlor-phenyldiazonium-tetrafluoroborat (Farbbildner)
12 ml Wasser

wird eine Beschichtungsmasse hergestellt und mit 0,13 mm Naßfilmdicke auf eine 0,2 mm starke durchsichtige Polycarbonatfolie beschichet und 15 Minuten bei 50°C getrocknet.

b) Herstellung des erfindungsgemäßen Reagenzfilms 11

aus
10 mmol/l 2,5-Di-Chlor-phenyldiazonium-tetrafluoroborat in 0,6% Xanthan-Gum in Wasser
wird eine Beschichtungsmasse hergestellt und mit 0,1 mm Naßfilmdicke auf eine 0,1 mm dicke durchsichtige Polycarbonatfolie (als Deckfolie 10) beschichtet und 15 Minuten bei 50°C getrocknet.
Der Reagenzträger 9 ist bei diesem Test nicht vorhanden.

c) Herstellung der Reagenzschicht 6 (Vorreagenz zur Freisetzung des konjugierten Bilirubins)

500 mmol/l Dyphyllin in Wasser werden auf Langfaserpapier getränkt und 10 Minuten bei 50°C getrocknet.

Messung mit einem Reflotron-Gerät mit Bilirubin-haltigen Seren nach einer Minute Reaktionszeit.

| **Gehalt**<br>**(mg/dl)** | **% Remission**<br>**Kieselgur-Film**<br>**(a)** | **% Remission**<br>**erfindungsgemäßer**<br>**Testträger (b)** |
|---|---|---|
| 1 | 60 | 62,5 |
| 2 | 58,5 | 58 |
| 4 | 56 | 52 |
| 8 | 52 | 44,5 |
| 12 | 48,5 | 39,5 |
| 20 | 45,5 | 32,5 |

Man erkennt, daß der Remissionshub beim erfindungsgemäßen Testträger etwa doppelt so hoch ist, wie bei Verwendung eines Reagenzfilms gemäß dem Stand der Technik. Bei gegebener Meßgenauigkeit des Auswertegerätes ergibt sich daraus eine wesentliche Verbesserung der Genauigkeit der Analyse. Dieser Vorteil wirkt sich insbesondere im Bereich geringer Remissionen, in dem die Erfindung zu einer drastischen Verbesserung führt, besonders stark aus, weil in diesem Bereich eine geringe Änderung der Remission bei den üblichen Testen einer verhältnismäßig starken Änderung der entsprechenden Konzentration entspricht.

Auch bei diesem Beispiel wurden die verwendeten Schichten zusätzlich visuell beurteilt. Der Kieselgur-Film nach dem Stand der Technik zeigte eine undefinierte Verteilung der Reaktionsfarbe. Betrachtet man dem Meßbereich wie das Meßauge des Remissionsphotometers von oben durch die Deckfolie 10 der Klappe 8, ist nur eine blasse Färbung zu erkennen, die offensichtlich nur einen Bruchteil der tatsächlich insgesamt vorhandenen Reaktionsfarbe ausmacht.

Im Gegensatz hierzu ist beim erfindungsgemäßen Film eine gleichmäßig dunkle Einfärbung zu erkennen.

**Patentansprüche**

1. Mehrschichtiger Testträger (1) zur analytischen Bestimmung eines Bestandteils einer flüssigen Probe, insbesondere einer Körperflüssigkeit, mit einer ersten Tragschicht (2) und einer zweiten Tragschicht (10), von denen wenigstens eine durchsichtig ist, wobei auf die erste Tragschicht (2) eine flüssigkeitsabsorbierende Schicht (3) und auf die zweite Tragschicht (10) eine mindestens ein Reagenz enthaltende Reagenzschicht (11) aufgebracht ist und wobei die Tragschichten so angeordnet und befestigt sind, daß die von den Tragschichten abgewandten Oberflächen (3a, 11a) der flüssigkeitsabsorbierenden Schicht und der Reagenzschicht in Kontakt zueinander gebracht und gegeneinander gedrückt werden können, dadurch gekennzeichnet, daß die Reagenzschicht (11) in der Probenflüssigkeit im wesentlichen rückstandsfrei löslich ist und die Aufnahmefähigkeit der flüssigkeitsabsorbierenden Schicht (3) für die Probenflüssigkeit so groß ist, daß eine darin absorbierte Flüssigkeitsmenge ausreicht, um die Reagenzschicht rückstandsfrei zu lösen, so daß in der Reagenzschicht (11) enthaltene Substanzen aus der Reagenzschicht (11) in die flüssigkeitsabsorbierende Schicht (3) übertreten, wenn beide Schichten gegeneinander gedrückt werden und daß die flüssigkeitsabsorbierende Schicht (3) bezüglich ihrer optischen Absorptionseigenschaften und ihrer Schichtdicke so gewählt ist, daß sie vom Meßlicht eines üblichen remissionsphotometrischen Auswertegerätes durchdrungen wird und daß eine der Tragschichten (2) für das Meßlicht diffus reflektierend ist.

2. Testträger nach Anspruch 1, dadurch gekennzeichnet, daß die Reagenzschicht (11) höchstens halb so dick wie die flüssigkeitsabsorbierende Schicht (3) ist.

3. Testträger nach Anspruch 1, dadurch gekennzeichnet, daß der Filmbildner ein Binde- oder Quellmittel oder Verdicker ist.

4. Testträger nach Anspruch 1, dadurch gekennzeichnet, daß die Reagenzschicht (11) als Film aus einem hochmolekularen, polymeren, reversibel in Wasser löslichen Material ausgebildet ist.

5. Testträger nach Anspruch 1, dadurch gekennzeichnet, daß die absorbierende Schicht (3) eine größere Flächenausdehnung als die Reagenzschicht (11) hat, so daß sie im zusammengedrückten Zustand der Schichten nicht vollständig von der Reagenzschicht (11) bedeckt ist und daß die flüssigkeitsabsorbierende Schicht (3) Kapillartransporteigenschaften in Richtung ihrer Flächenausdehnung hat.

EP 0 262 445 B1

6. Testträger nach Anspruch 1, dadurch gekennzeichnet, daß eine im zusammengedrückten Zustand der Reagenzschicht (11) und der flüssigkeitsabsorbierenden Schicht (3) zwischen diesen Schichten angeordneter Reagenzträger (9) mit einer für die Probenflüssigkeit offenen Netz- oder Gewebestruktur vorgesehen ist, der mit mindestens ein weiteres Reagenz trägt.

7. Testträger nach Anspruch 1, dadurch gekennzeichnet, daß die erste Tragschicht (2) eine steife Folie und die zweite Tragschicht (10) eine flexible Folie ist.

8. Testträger nach Anspruch 7, dadurch gekennzeichnet, daß die steife Folie 200 – 600 μm und die flexible 100 – 250 μm stark ist.

9. Testträger nach Anspruch 1, dadurch gekennzeichnet, daß die zweite Tragschicht (10) eine gerade Kante aufweist und an dieser Kante klappenartig mit der ersten Tragschicht verbunden ist.

10. Testträger gemäß Anspruch 3, dadurch gekennzeichnet, daß der Filmbildner Xanthan ist.

**Claims**

1. A multi-layer test carrier (1) for the analytical determination of a component in a liquid sample, especially in a body fluid, with a first carrier layer (2) and a second carrier layer (10), at least one of which is transparent, whereby a liquid-absorbing layer (3) is applied to the first carrier layer (2) and a reagent layer (11) containing at least one reagent is applied to the second carrier layer (10) and whereby the carrier layers are so arranged and fixed that the surfaces (3a, 11a) of the liquid-absorbing layer and of the reagent layer facing away from the carrier layers can be brought into contact with one another and pressed against one another, characterised in that the reagent layer (11) is soluble substantially without residue in the sample liquid and the take-up ability of the liquid-absorbing layer (3) for the sample liquid is so great that an amount of liquid absorbed therein suffices in order to dissolve the reagent layer free of residues so that substances contained in the reagent layer (11) pass over from the reagent layer (11) into the liquid-absorbing layer (3) when the two layers are pressed against one another and that the liquid-absorbing layer (3) is, with regard to its optical absorption properties and its layer thickness, so chosen that it is penetrated by the measurement light of a conventional remission photometric evaluation device and that one of the carrier layers (2) is diffusely reflecting for the measurement light.

2. Test carrier according to claim 1, characterised in that the reagent layer (11) is at most half as thick as the liquid-absorbing layer (3).

3. Test carrier according to claim 1, characterised in that the film former is a binding or swelling agent or a thickening agent.

4. Test carrier according to claim 1, characterised in that the reagent layer (11) is made as a film of a high molecular, polymeric material reversibly soluble in water.

5. Test carrier according to claim 1, characterised in that the absorbing layer (3) has a greater planar extension than the reagent layer (11) so that, in the pressed-together state of the layers, it is not fully covered by the reagent layer (11) and that the liquid-absorbing layer (3) has capillary transport properties in the direction of its planar extension.

6. Test carrier according to claim 1, characterised in that, in the pressed-together state of the reagent layer (11) and of the liquid-absorbing layer (3), a reagent carrier (9) arranged between these layers is provided with an open mesh or fabric structure for the sample liquid which contains at least one further reagent.

7. Test carrier according to claim 1, characterised in that the first carrier layer (2) is a stiff film and the second carrier layer (10) is a flexible film.

8. Test carrier according to claim 7, characterised in that the stiff film has a thickness of 200 to 600 μm and the flexible one a thickness of from 100 to 250 μm.

9. Test carrier according to claim 1, characterised in that the second carrier layer (10) has a straight edge and is attached flaplike on this edge to the first carrier layer.

10. Test carrier according to claim 3, characterised in that the film former is xanthan.

**Revendications**

1. Support de test multicouche (1) pour la détermination analytique d'un composant d'un échantillon liquide, en particulier d'un fluide corporel, avec une première couche de support (2) et une seconde couche de support (10), dont au moins l'une est transparente, une couche (3) absorbant les liquides étant appliquée sur la première couche de support (2) et une couche réactive (11), contenant au moins un réactif, étant appliquée sur la seconde couche de support (10), et les couches de support étant disposées et fixées de telle façon que les faces (3a, 11a) opposées aux couches de support de la couche absorbant les liquides et de la couche réactive soient en contact entre elles et puissent être pressées l'une contre l'autre, caractérisé en ce que la couche réactive (11) est soluble dans le liquide échantillon pratiquement sans résidu et que la capacité d'absorption de la couche (3) absorbant les liquides, pour le liquide échantillon, est d'une grandeur telle que la quantité de liquide qu'elle absorbe suffit pour dissoudre sans résidu la couche réactive, de sorte que les substances contenues dans la couche réactive (11) passent de la couche réactive (11) dans la couche (3) absorbant les liquides, lorsque les deux couches sont pressées l'une contre l'autre et que la couche (3) absorbant les liquides est choisie, au point de vue de ses propriétés d'absorption optique et de l'épaisseur de la couche, de façon que la lumière de mesure d'un apareil usuel d'évaluation par photométrie de réflexion puisse la traverser, et l'une des couches de support (2) réfléchit de façon diffuse la lumière de mesure.

2. Support de test selon la revendication 1, caractérisé en ce que la couche réactive (11) présente une épaisseur qui est au maximum la moitié de l'épaisseur de la couche (3) absorbant les liquides.

3. Support de test selon la revendication 1, caractérisé en ce que le formateur de film est un agent liant ou gonflant ou un épaississant.

4. Support de test selon la revendication 1, caractérisé en ce que la couche réactive (11) est constituée d'un film en un matériau polymère, hydrosoluble de façon réversible, de masse moléculaire élevée.

5. Support de test selon la revendication 1, caractérisé en ce que la couche absorbante (3) présente une superficie plus grande que la couche réactive (11), de sorte qu'à l'état pressé l'une contre l'autre, la couche absorbante n'est pas complètement recouverte par la couche réactive (11) et que la couche (3) absorbant les liquides présente des propriétés de transport capillaire dans le sens de sa superficie.

6. Support de test selon la revendication 1, caractérisé en ce qu'un support de réactif (9) disposé entre la couche réactive (11) et la couche (3) absorbant les liquides, à l'état ou ces couches sont pressées l'une contre l'autre, est prévu avec une structure de filet ou de tissu ouverte pour le liquide échantillon, et qui porte au moins un autre réactif.

7. Support de test selon la revendication 1, caractérisé en ce que la première couche de support (2) est une feuille rigide et que la seconde couche de support (10) est une feuille souple.

8. Support de test selon la revendication 7, caractérisé en ce que la feuille rigide est d'une épaisseur de 200 à 600 µm et que la feuille souple est d'une épaisseur de 100 à 250 µm.

9. Support de test selon la revendication 1, caractérisé en ce que la seconde couche de support (10) présente une arête droite et que suivant cette arête, elle est peut être rabattue sur la première couche de support.

10. Support de test selon la revendication 3, caractérisé en ce que le formateur de film est le xanthane.

EP 0 262 445 B1

Fig 1

Fig 2